# EUROPEAN PATENT SPECIFICATION

(11) **EP 1 884 208 B1**
(45) Date of publication and mention of the grant of the patent: **25.02.2015**
(21) Application number: 06756427.8
(22) Date of filing: 22.05.2006
(51) Int. Cl.: A61B 17/12

(54) **MEDICAL WIRE**
MEDIZINISCHER DRAHT
FIL MEDICAL

(30) Priority: 24.05.2005 JP 2005151203
(43) Date of publication of application: 06.02.2008
(73) Proprietor: Kaneka Corporation, Osaka (JP)
(72) Inventor: TAKATA, Hironori, Settsu-shi, Osaka 5660072 (JP); ISHIDA, Jiro, Settsu-shi, Osaka 5660072 (JP)
(74) Representative: Gille Hrabal
(86) International application number: PCT/JP2006/310121
(87) International publication number: WO 2006/126474

(56) References cited:
- WO-A1-01/58366
- JP-A- 07 265 431
- JP-A- 10 094 542
- JP-B2- 2 501 389
- JP-B2- 3 029 993
- US-A- 4 748 986
- US-A- 4 748 986
- US-A1- 2004 199 175

## Description

### Technical Field

The present invention relates to a medical wire for indwelling a member at a specified position in a biological body.

### Background Art

As a treatment method of low invasiveness to aneurysm and the like, vascular embolization is currently known, which is a procedure for indwelling in the biological body a member made up of a metal such as platinum not affecting the body adversely.

In this procedure, an indwelling member connected to a wire for delivery is passed into a catheter; after guiding the member to a desired position in the body while being X-rayed with an X-ray contrast imaging device, the member is conveyed to a target position by handling of the wire and separated. In regard to the procedure for separating the indwelling member, there are mechanical and electrical types. For example, in the electrical type, an electrical current is supplied from outside to between an electrically conductive wire and a counter electrode connected to the indwelling member, thereby decomposing and melting down a connection member, and separating the indwelling member. As shown in Japanese Published Patent No. Hei7-265431, a device for vascular blockage using a thermally soluble member composed of hot-meltable polyvinyl alcohol as the connection member between the electrically conductive wire and the indwelling member is characterized by a shortened operating time and less burden upon patients and doctors in as much as the connection member is instantly hot-melted to render separation from the indwelling member upon electrode heating by means of a high-frequency, electrical current supplied to a tip of the electrically conductive wire serving as the heating electrode and the counter electrode.

Precautions in either of the above-mentioned procedures are not to injure a weak wall of aneurysm while delivering the indwelling member. It is indispensable not to cause injury due to a delivery implement, as well as the indwelling member. However, in the device for vascular blockage mentioned above having the conventional constitution, especially the connection member, there was a problem in which at the junction of the connection member, the indwelling member, and the delivery implement, the wall of aneurysm is injured due to its rigidity, as a catheter arranged inside the aneurysm moves and further as the catheter moves inside the aneurysm.

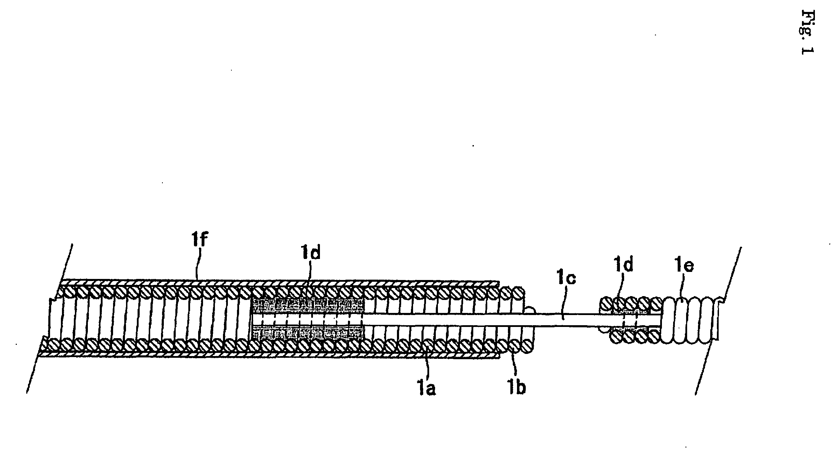

Specifically, Fig. 1 is an enlarged, sectional view of the connection between an electrically conductive pusher wire and the indwelling member in an example of the conventional medical wire. As shown in Fig. 1, in this medical wire, at a tip of the electrically conductive pusher wire 1a, there is formed an electrode unit 1b in a coil shape, inside of which a proximal end of a rod-shape thermally soluble connection member 1c made up of polyvinyl alcohol is inserted and fixed by an adhesive 1d, while the tip of the thermally soluble connection member 1c is inserted into a coil forming an indwelling member 1e and likewise fixed by the adhesive 1d. Reference character 1f represents a fluorine included resin coating which forms an insulating surface provided at a portion other than the tip of the electrically conductive pusher wire and the proximal end.

Nonetheless, in such constitution, since the thermally soluble connection member 1c and a coil wire of the electrically conductive pusher wire 1a are bonded inside the fluorine included resin coating, a neighboring coil wire section is mutually linked solidly, thus increasing shear force in a radial direction. As a result, flexibility of the electrically conductive pusher wire is obstructed.

US 2004/199175 A discloses a medical wire comprising a forward end member formed on an electrically conductive wire in a coil shape with a proximal end and a distal end, an electrically conductive pusher wire connected to the proximal end of the forward end member, and an indwelling member connected via an electrically soluble connection member to the distal end of the forward end member, whereby said member wire separating from the indewelling member as a result of an electrolyse of the connection member by an electrical current.

A portion of the coil wire section of the forward end member is not in intimate contact with a neighboring coil wire section. Moreover, the coil wire of the forward end member has two different pitches and the medical wire is provided with a safety wire positioned inside the coil winding of said forward end member, extending between the proximal end and the distal end by connecting both ends.

### DISCLOSURE OF THE INVENTION

### Problems to Be Solved by the Invention

In view of the above-mentioned circumstances, the present invention purports to provide a medical wire for delivering a flexible indwelling member of excellent safety.

### Means of Solving the Problems

To achieve the above-mentioned purpose, it is an object of the present invention to provide a medical wire which comprises a forward end member being formed of an electrically conductive material in a coil shape with a proximal end and a distal end, an electrically conductive pusher wire, and an indwelling member which is connected via a thermally soluble connection member to the distal end of such forward end member, the medical wire separating the indwelling member as a result of heating the connection member by an electrical current, characterized in that there is included a portion of the coil wire section of the forward end member not in intimate contact with an neighboring coil wire section, that the coil wire of the forward end member has at least more than two different pitches, and that said medical wire is provided with a safety wire positioned inside coil winding of said forward end member, extending between the proximal end and the distal end, and connecting both ends.

It is another object of the present invention to provide the medical wire in which the safety wire is either a round wire or a flat wire.

Further, the present invention provides the medical wire in which one end of the safety wire is welded, brazed or bonded to at least one point on an inner circumference of the forward end member, while the other end thereof is welded, brazed or bonded to both or one of the proximal end of the forward end member and the electrically conductive pusher wire.

Still further, the present invention provides the medical wire in which the forward end member and the safety wire include a biocompatible metal.

Furthermore, the present invention provides the medical wire in which the forward end member and the safety wire include at least one kind of metal selected from a group composed of platinum, palladium, gold, tungsten, tantalum, iridium, stainless steel alloys, hyperelastic alloys, and alloys of these metals.

Further, the present invention provides the medical wire which is constituted by the forward end member having an electrode unit (first section) for hot-melting the thermally soluble connection member by the electrical current, a portion (second section) in which one end of the safety wire is connected to the inner circumference of the coil wire making up the forward end member, and a portion (third section) to which the proximal end of the thermally soluble connection member is bonded and fixed.

Further, the present invention provides the medical wire in which the mutually neighboring coil wires constituting the first section of the forward end member are mutually in intimate contact.

Further, the present invention provides the medical wire in which the thermally soluble connection member is in intimate contact with the inner circumference of the coil wire making up the first section of the forward end member.

Further, the present invention provides the medical wire in which the pitch of the coil wire in the second section of the forward end member is set larger than the pitch of the coil wire in the third section.

Further, the present invention provides the medical wire in which the pitch of the coil wire in the third section is more than 110% of the pitch when the neighboring coil wire in intimate contact is set as 100%.

### Effects of the Invention

According to the medical wire of the present invention, in the forward end member attached to the tip of the electrically conductive pusher wire, a portion in which the coil wire of the forward end member is not in intimate contact with the neighboring coil wire is not included and the coil wire of the forward end member has at least more than two different pitches, whereby flexibility is not flawed at the time of connecting to the connection member as a result of the coil wire of the forward end member having at least two different pitches. Moreover, according to the present invention, by suppressing forward end member growth with the safety wire and thus making it difficult for the forward end member to deform or break in case of delivering the indwelling member, it is possible to deliver the indwelling member that excels in safety.

### Brief Explanation of the Drawings

Fig. 1 shows an enlarged section of a conventional medical wire,
Fig. 2 shows an enlarged section of a medical wire according to the present invention,
Fig. 3 shows a procedure for using the medical wire according to the present invention,
Fig. 4 shows a configuration of electric circuitry of the medical wire according to the present invention, and
Fig. 5 shows a diagram of measurement of flexibility of the medical wire according to the present invention.

### Explanation of Reference Characters

- 1a: Electrically conductive pusher wire,
- 1b: Electrode unit,
- 1c: Thermally soluble connection member,
- 1d: Adhesive,
- 1e: Indwelling member,
- 1f: Fluorine included resin coating,
- 2a: Electrically conductive pusher wire,
- 2b: Forward end member,
- 2c: Thermally soluble connection member,
- 2d: Safety wire,
- 2e: Indwelling member,
- 2f: Coating,
- 2g: Inner circumference of forward end member,
- 2h: Proximal end of forward end member,
- 2i: Adhesive,
- 2j: Electrode unit,
- 3a: Aneurysm,
- 3b: Catheter,
- 3c: Electrode unit
- 3d: Holder
- 3e: Indwelling member,
- 3f: Thermally soluble connection member,
- 3g: Body
- 4a: Proximal end of electrically conductive pusher wire,
- 4b: Counter electrode plate
- 4c: Electrically conductive pusher wire,
- 4d: Indwelling member,
- 4e: Electrical power unit,
- 4f: Electrode unit,
- 4g: Thermally soluble connection member, and
- 4h: Catheter.

### Best Mode for Carrying out the Invention

Referring to the drawings, an embodiment of the present invention will be described below with respect to embolization of aneurysm.

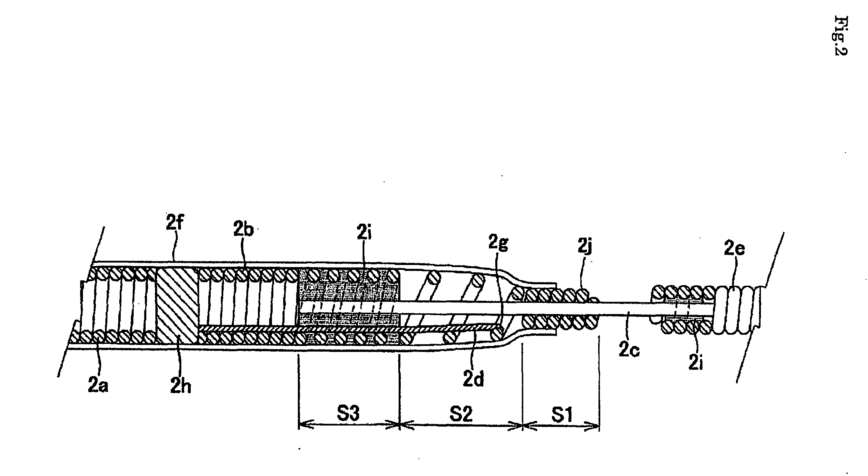

Fig. 2 is an enlarged sectional view of a connection between an electrically conductive pusher wire and an indwelling member in an example of a medical wire according to the present invention. As shown in Fig. 2, the medical wire of the present invention consists of an indwelling member 2e composed of a metal not adversely affecting a biological body, an electrically conductive pusher wire 2a capable of delivering the indwelling member 2e to a targeted region, a thermally soluble connection member 2c connecting the indwelling member 2e and a forward end member 2b attached to a tip of the electrically conductive pusher wire 2a, and a safety wire 2d positioned inside coil winding of the forward end member, extending between a proximal end and a distal end, and connecting both ends.

As the indwelling member 2e, any indwelling member used for vascular embolization may be used. As a shape of the indwelling member 2e, various shapes such as a spiral shape, an S-shape, a spiral shape with its radius changing, and further a shape with its primary shape and secondary shape given may be available. Particularly from the standpoint of sufficient actual achievements in arterial treatment, a secondary coil shape in which the primary shape is a circular spiral to which a circular secondary spiral is provided is desirable.

As a material of wiring that makes up the coil, a biocompatible metal, for example, platinum, tungsten, gold, tantalum, iridium, stainless steel alloys, and alloys of these metals may be used. Especially, in terms of actual achievements of arterial treatment, platinum alloys, and further, alloys of platinum and tungsten are desirable. Note that a sectional shape of the wire is not limited to a circular form, but that a variety of shapes such as ellipse, square, and triangle are applicable. Furthermore, the primary coil having a secondary coil shape or a tertiary shape with its proximal side processed roundly so as not to impair the biological body is best suited.

As the shape of the electrically conductive pusher wire 2a, the spiral shape, the S-shape, the spiral shape with its radius changing may be cited. In a preferred embodiment, the shape of the electrically conductive pusher wire 2a is of such construction that the tip of a stainless steel alloy-made wire section in a tapered manner is covered with a stainless steel alloy and a platinum alloy coil and fixed, the tip having an inner hole due to the coil shape. The outer diameter of the tip of over 0.25 mm and the inner diameter of over 0.12 mm are suitable. Further, the outer diameter of the tip is preferably over 0.25 mm and below 0.45 mm. The inner diameter of the tip is more preferably over 0.12 mm and below 0.20 mm.

To the tip of the electrically conductive pusher wire 2a there is connected the forward end member 2b. The forward end member 2b includes a portion of the coil wire not in intimate contact with the neighboring coil wire section, the coil wire having at least over two different pitches. Such construction contributes to making the medical wire of high flexibility.

Now, in the present invention, that the coil wire constituting the forward end member is not in intimate contact with the neighboring coil wire section indicates the surfaces of mutually neighboring coil wires not in completely intimate contact with each other, including a state in which some portion is not in intimate contact. Further, in the present invention, the pitch shows a pitch distance with one turn of the neighboring coil wire, and "having at least over two different pitches" means that a pitch distance with one turn is not constant and that the coil is wound in more than two different distances.

A spiral shape, an S-shape, or a spiral shape with its radius changing may be cited for the shape of the forward end member 2b connected to the tip of the electrically conductive pusher wire 2a. In the preferred embodiment, the shape of the forward end member 2b connected to the tip of the electrically conductive pusher wire 2a is the electrically conductive wire formed into the coil shape. As the material of the wire, at least one kind of metal selected from a group consisting of a biocompatible metal, for example, platinum, palladium, tungsten, gold, tantalum, iridium, stainless steel alloys, hyperelastic alloys, and alloys of these metals and any alloy selected from these metals may be used. Particularly, use of stainless steel alloys is preferable. The outer diameter and the inner diameter are preferably equivalent to the outer diameter and the inner diameter of the tip of the electrically conductive pusher wire 2a. This portion is preferably such as not to create a step at the connection with the electrically conductive pusher wire 2a.

This is for reducing the extent of being held up due to this step when delivering and preventing the wire from deforming at the catheter's inner hole and lead port.

The forward end member is, for example, as shown in Fig. 2, made up of an electrode unit 2j (first section) for hot-melting the thermally soluble connection member 2c with an electric current, a portion (second section) in which one end of the safety wire 2d is connected to the inner circumference 2g of the coil wire making up the forward end member, and a portion (third section) to which the proximal end of the thermally soluble connection member 2c is bonded and fixed.

The first section (S1) of the forward end member is used as the electrode for hot-melting with an electric current the thermally soluble connection member 2c arranged on the inner circumference of the coil wire making up the forward end member while its number of turns of wire and pitch are selected arbitrarily from impedance necessary for heat generation.

As the thermally soluble connection member 2c, a material having sufficiently large rigidity or mechanical strength at the body temperature, e.g., 35 to 37°C, with its fusing point under 100°C may be cited. From the standpoint of ability to separate by supplying a high-frequency electric current for an extremely short period of 1 to 3 seconds, a material containing polyvinyl polymer or co-polymer is preferable as the thermally soluble connection member 2c.

Further, from the standpoint of ability to operate such as generating heat in a short period of time, it is preferable for the neighboring coil wire constituting the first section of the forward end member to be mutually in intimate contact.

Further, from the standpoint of good thermal conductivity, the thermally soluble connection member 2c is preferably in intimate contact with the inner circumference of the coil wire constituting the first section of the forward end member.

While the second section (S2) of the forward end member is used to connect one end of the safety wire 2d to the inner circumference 2g of the coil wire making up the forward end member, it is also used to suppress growth of the forward end member so as to suppress deformation or breaking of the forward end member and further to secure flexibility of the medical wire. A distance between its neighboring coil wires is suitably set at more than 400% if it is set at 100% in the case of neighboring coil wires in intimate contact, so that it is desirable for processing such as welding to be made possible by using a clearance gap with the neighboring coil wires. The distance between the neighboring coil wires is preferably over 400% and under 500%.

Further as a method of connecting the safety wire 2d, there may be available welding, brazing and bonding, one end thereof being connected to the inner circumference 2g of the forward end member while the other end being connected to both or one of the proximal end 2h and the electrically conductive pusher wire 2a. Any long member may be used for the safety wire 2d so long as the forward end member is capable of suppressing growth in excess of a prescribed length. The safety wire 2d extending between the proximal end and the distal end and connecting both ends is used for the purpose of arresting growth of the forward end member. If such safety wire 2d is not available, the forward end member may possibly deform or break down at the time of delivery. Namely, if a pitch is widened to secure flexibility of the forward end member, it becomes easy for an end of the catheter to be caught in the clearance gap between wires, so that once the end of the catheter is caught, the pitch widens further with a possibility that the wire may break depending on the condition. Disposition of the safety wire 2d will avoid such risk.

As the material of the safety wire, there may be employed at least one kind of metal selected from a group consisting of platinum, palladium, tungsten, gold, tantalum, iridium, stainless steel alloys, hyperelastic alloys, and any alloy selected from these metals. Use of stainless steel alloys is especially desirable.

Further, the pitch of the coil wire in the second section is preferably set larger than the pitch of the coil wire of the third section to be mentioned later. In this case, there is an advantage in that since it is difficult for the adhesive injected into the third section to run into the second section, the first section on the further tip side will not be contaminated with the adhesive. This means, however, that when the electrode unit 2j and the thermally soluble connection member 2c are bonded by the adhesive in the first section, even though the electrode unit 2j is heated, it will not result in separating the indwelling member 2e.

The third section S3 of the forward end member is employed for bonding and fixing the proximal end of the thermally soluble connection member. Here it is used to prevent mutually neighboring wires from being solidly linked by the adhesive 2i and as a liquid pool for preventing the adhesive 2i from running over the coil wire to the distal end due to capillary action, its pitch being preferably over 110% if the pitch when the neighboring coil wires are in intimate contact is set at 100%. Over 110% and below 140% are desirable.

While the adhesive 2i may serve to fill the inside of the coil wire, preferably it should be used between coil wires from the standpoint of securing flexibility of the medical wire.

The tip side of the thermally soluble connection member 2c is inserted into an inner hole of the indwelling member 2e, bonded and fixed. The outer diameter of the thermally soluble connection member 2c is over 0.1 mm and its total length is over 1 mm. Its length of insertion into the inner hole of the forward end member 2b and the inner hole of the indwelling member 2e is preferably over 0.3 mm. Further, the outer diameter of the thermally soluble connection member 2c is preferably over 0.10 mm and below 0.18 mm. Its total length is preferably over 1 mm and below 3 mm. Its length of insertion into the inner hole of the forward end member 2b and the inner hole of the indwelling member 2e is preferably over 0.3 mm and below 0.5 mm.

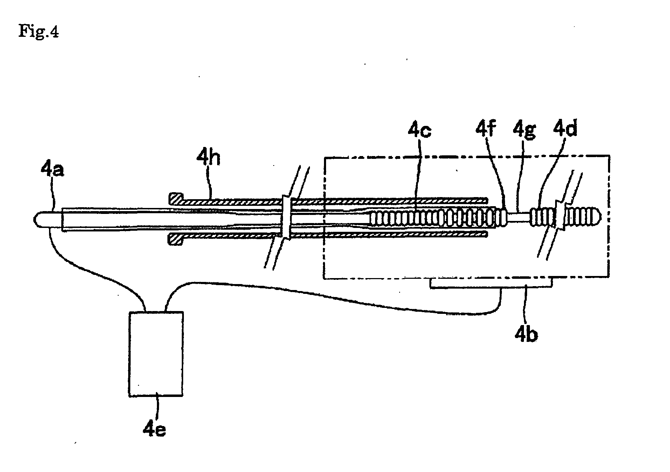

Still further, a coating may be provided at a portion other than the electrode unit 2j and the proximal end (Fig. 4-4a) of the forward end member. By setting up such coating (2f of Fig. 2), there is an advantage of improvement of slidability of the medical wire inside the catheter. As the coating, a resin forming an insulating surface, e.g., fluorine included resin, is desirable.

Moreover, the proximal end 2h of the forward end member 2b connected to the coil wire making up the electrically conductive pusher wire 2a is of such construction that, for example, after aligning the tip of the electrically conductive pusher wire 2a and the proximal end of the forward end member 2b on the same axis line, it is, for example, bonded by laser welding. In addition to laser welding, a step such as brazing or soldering may be employed.

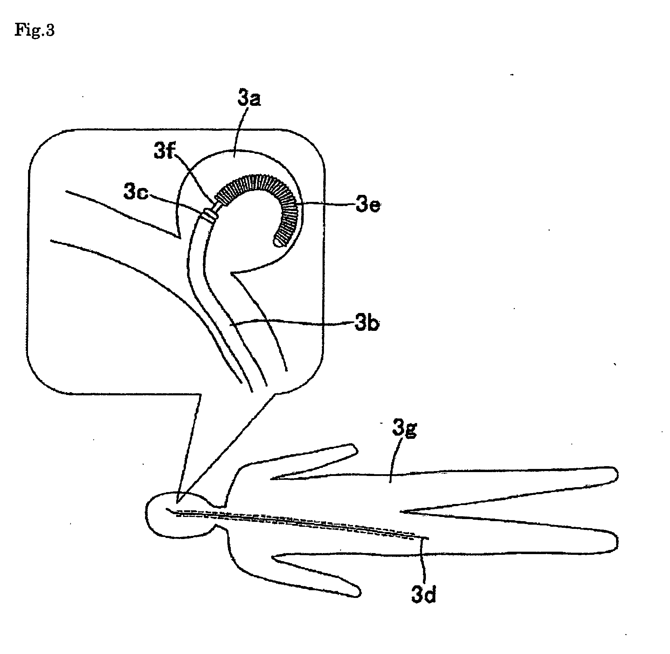

The medical wire of the foregoing construction may be used as follows. Namely, as shown in Fig. 3, the medical wire is introduced into the catheter 3b disposed in the biological body 3g to which the indwelling member 3e is to be applied, and the indwelling member 3e is guided to aneurysm 3a while sighting through the x-ray. This operation is conducted while gripping a holder 3d on this side (near side) where the insulating surface of the electrically conductive pusher wire is formed. And from the tip of the catheter 3b, the electrode unit 3c of the forward end member and a portion on the farther side, that is, the electrode unit 3c, the thermally soluble connection member 3f and the indwelling member 3e jut out, thus creating a condition in which the indwelling member 3e has been introduced into the aneurysm 3a.

On the other hand, as shown in Fig. 4, the electrical power unit 4e is connected to between the proximal end 4a of the electrically conductive pusher wire 4c and the counter electrode plate 4b provided in contact with the body surface of the biological body, whereby an electrical circuit is constituted between the electrically conductive pusher wire 4c and the counter electrode plate 4b. And while in the condition of the indwelling member 4d being inserted inside the catheter 4b into the aneurysm (for example, Fig. 3), a heating electric current from the power unit 4e is supplied to the electrically conductive pusher wire 4c, so that the electrode unit 4f generates heat to make hot the thermally soluble connection member 4g, melting down the thermally soluble connection member 4g, which has been swollen with water and the like in advance, with the resultant separation of the indwelling member 4d.

Nevertheless, when indwelling the indwelling member in the aneurysm as mentioned above, the indwelling member is delivered though the catheter that was introduced into the aneurysm. But in case rigidity of the pusher wire is high, rather than delivering the indwelling member along the bending of the catheter, there is a possibility of the catheter itself being stretched in a straight line due to the rigidity of the pusher wire. Further, there is a risk of injuring the aneurysm wall as the catheter moves inside the aneurysm. Hence, according to the construction having a forward end member as in the case of the present invention, due to the flexibility of the forward end member, the aneurysm wall will not be injured, so that delivery of the indwelling member may be performed safely.

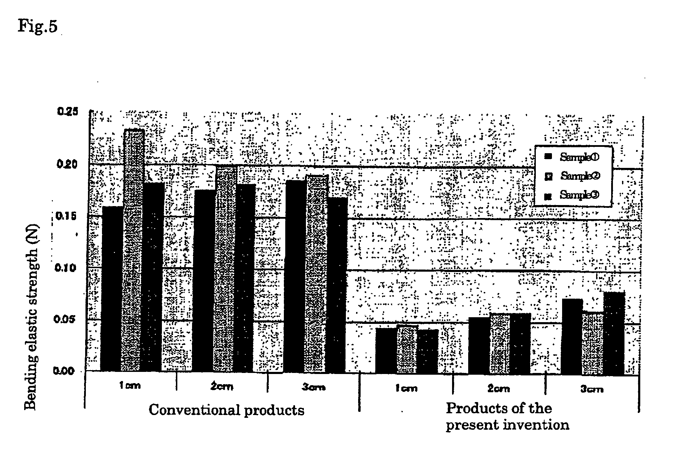

Flexibility of the tip of the pusher wire was measured to confirm the results of the present invention and a comparison was made to the conventional pusher wire. Fig. 5 shows the results of the comparison. Three pieces each of the conventional pusher wire and the pusher wire of the present invention (samples 1-3) were used. Compression tests of the samples were conducted on the positions of 1cm, 2cm, and 3cm from the tip of the pusher wire to the other end to measure their bending elastic strength (as testing procedures, a load was measured when the indenter was pushed in by 2 mm at a speed of 50 mm/min). As shown in Fig. 5, the products of the present invention show the bending elastic strength of approx. 20% of the conventional products at the measuring point of 1cm, approx. 30% of the conventional products at the measuring point of 2cm, and approx. 40% of the conventional products at the measuring point of 3cm. Therefore, it is seen that according to the construction having such forward end member as the present invention, elasticity of the forward end member improves to enable safe delivery of the indwelling member.

Note that in the above-mentioned embodiment, description was made by using the procedures of coil embolization of aneurysm as an example. It is not limited to this application but may be used for general treatment to separate the indwelling member from the electrically conductive wire.

### INDUSTRIAL APPLICABILITY

The medical wire of the present invention is applicable for general treatment to separate the indwelling member from the electrically conductive wire. Above all, it can be safely used for treatment of diseases of low invasiveness to aneurysm.

## Claims

1. A medical wire comprising a forward end member (2b) being formed of an electrically conductive wire in a coil shape with a proximal end (2h) and a distal end, an electrically conductive pusher wire (2a) connected to the proximal end (2h) of the forward end member (2b), and an indwelling member (2e) connected via a thermally soluble connection member (2c) to the distal end of the forward end member (2b), said medical wire separating said indwelling member as a result of heating said connection member by an electrical current, a part of the coil wire section of said forward end member being provided not to be in intimate contact with an neighbouring coil wire section In a cross-sectional view, the coil wire of said forward end member having at least more than two different pitches, and said medical wire being provided with a safety wire (2d) positioned inside coil winding of said forward end member, extending between the proximal end (2h) and the distal end, and connecting both ends, **characterized In that** the distal end of the safety wire is connected to an inner circumference of the coil wire constituting the forward end member.

2. The medical wire according to claim 1, **characterized In that** one end of the safety wire is a distal end of the safety wire.

3. The medical wire according to claim 1 or 2, **characterized in that** a proximal end of the safety wire is bonded to both or one of the proximal end (2h) of said forward end member and the electrically conductive pusher wire (2a).

4. The medical wire according to anyone of claims 1 to 3, **characterised in that** the connection of the distal end of the safety wire to the inner circumference (2g) of the coil wire constituting the forward end member (2b) Is welding, braising or bonding.

5. The medical wire according to any one of claims 1 to 4, **characterized in that** said safety wire is a round wire or a square wire.

6. The medical wire according to any one of claims 1 to 5, **characterized in that** said forward end member and the safety wire (2d) include a biocompatible metal.

7. The medical wire according to any one of claims 1 to 6, **characterized in that** said forward end member and said safety wire include at least one kind of metal selected from a group comprised of platinum, palladium, gold, tungsten, tantalum, iridium, stainless steel alloys, hyperelastic alloys, and alloys of these metals.

8. The medical wire according to any one of claims 1 to 7, **characterized in that** said forward end member includes an electrode unit (2j) for hot-melting said thermally soluble connection member by an electrical current, and a portion to which the proximal end (2h) of said thermally soluble connection member (2c) Is bonded and fixed.

9. The medical wire according to claim 6, **characterized by** the neighbouring coil wires making up the electrode unit (2j) of the forward end member (2b) which are mutually in intimate contact.

10. The medical wire according to claim 9, **characterized by** said thermally soluble connection member in intimate contact with the inner circumference (2g) of the coil wire making up the electrode unit (2j) of the forward end member (2b).

11. The medical wire according to any one of claims 1 to 10, **characterized in that** a pitch of the coil wire of a second section where the distal end of the safety wire is connected to the inner circumference (2g) of the coil wire making up the forward end member (2b), is set larger than the pitch of the coil wire of a third section.

12. The medical wire according to any one of claims 8 to 11, **characterized in that** the pitch of the coil wire of the forward end member (2b) in a portion to which the proximal end (2h) of said thermally soluble connection member (2c) is bonded and fixed, is over 110% if the neighbouring coil wires in mutually intimate contact is set as 100%.

## Patentansprüche

1. Medizinischer Draht, welcher ein vorderes Endstück (2b), das aus einem elektrisch leitfähigen Draht in einer Spulenform mit einem proximalen Ende (2h) und einem distalen Ende gebildet wird, einen elektrisch leitfähigen Schiebedraht (2a), der mit dem proximalen Ende (2h) des vorderen Endstücks (2b) verbunden ist, und ein Einsatzteil (2e), das über ein thermisch lösbares Verbindungselement (2c) mit dem distalen Ende des vorderen Endstücks (2b) verbunden ist, umfasst, wobei der medizinische Draht das Einsatz durch das Erhitzen des Verbindungselements mittels eines elektrischen Stroms trennt, wobei ein Teil des Spulendrahtabschnitts des vorderen Endstücks so bereitgestellt wird, dass es bei querschnittlicher Betrachtung in keinem direkten Kontakt mit einem benachbarten Spulendrahtabschnitt ist, wobei der Spulendraht des vorderen Endstücks mindestens mehr als zwei verschiedene Windungsabstände aufweist, und der medizinische Draht mit einem Sicherheitsdraht (2d) bereitgestellt wird, der innerhalb der Spulenwindungen des vorderen Endstücks angeordnet ist, zwischen dem proximalen Ende (2h) und dem distalen Ende verläuft und beide Enden verbindet, **dadurch gekennzeichnet, dass** das distale Ende des Sicherheitsdrahts mit einem inneren Umfang des Spulendrahts, der das vordere Endstück bildet, verbunden ist.

2. Der medizinische Draht nach Anspruch 1, **dadurch gekennzeichnet, dass** ein Ende des Sicherheitsdrahtes ein distales Ende des Sicherheitsdrahtes ist.

3. Der medizinische Draht nach Anspruch 1 oder 2, **dadurch gekennzeichnet, dass** ein proximales Ende des Sicherheitsdrahtes an beide oder ein Ende des proximalen Endes (2h) des vorderen Endstücks und dem elektrisch leitfähigen Schiebedraht (2a) gebunden ist.

4. Der medizinische Draht nach einem der Ansprüche 1 bis 3, **dadurch gekennzeichnet, dass** die Verbindung des distalen Endes des Sicherheitsdrahtes zum inneren Umfang (2g) des Spulendrahtes, der das vordere Endstück (2b) bildet, Schweißen, Löten oder Kleben ist.

5. Der medizinische Draht nach einem der Ansprüche 1 bis 4, **dadurch gekennzeichnet, dass** der Sicherheitsdraht ein runder Draht oder ein Vierkantdraht ist.

6. Der medizinische Draht nach einem der Ansprüche 1 bis 5, **dadurch gekennzeichnet, dass** das vordere Endstück und der Sicherheitsdraht (2d) ein biokompatibles Metall enthalten.

7. Der medizinische Draht nach einem der Ansprüche 1 bis 6, **dadurch gekennzeichnet, dass** das vordere Endstück und der Sicherheitsdraht mindestens eine Art von Metall, ausgewählt aus der Gruppe, bestehend aus Platin, Palladium, Gold, Wolfram, Tantal, Iridium, rostfreie Stahllegierungen, hyperelastische Legierungen und Legierungen dieser Metalle enthalten.

8. Der medizinische Draht nach einem der Ansprüche 1 bis 7, **dadurch gekennzeichnet, dass** das vordere Endstück eine Elektrodeneinheit (2j) zum Heißschmelzen des thermisch lösbaren Verbindungselements durch einen elektrischen Strom und einen Abschnitt, an das das proximale Ende (2h) des thermisch lösbaren Verbindungselements (2c) gebunden und fixiert ist, umfasst.

9. Der medizinische Draht nach Anspruch 8, **gekennzeichnet durch** die benachbarten Spuldrähte, die die Elektrodeneinheit (2j) des vorderen Endstücks (2b) bilden, welche miteinander in direktem Kontakt stehen.

10. Der medizinische Draht nach Anspruch 9, **gekennzeichnet durch** das thermisch lösbare Verbindungselement in direktem Kontakt mit dem inneren Umfang (2g) des Spuldrahts, der die Elektrodeneinheit (2j) des vorderen Endstücks (2b) bildet.

11. Der medizinische Draht nach einem der Ansprüche 1 bis 10, **dadurch gekennzeichnet, dass** der Windungsabstand des Spuldrahtes eines zweiten Abschnitts, wo das distale Ende des Sicherheitsdrahtes mit dem inneren Umfang (2g) des Spuldrahtes, der das vordere Endstück (2b) bildet, verbunden ist, größer als der Windungsabstand des Spulendrahtes eines dritten Abschnitts festgesetzt wird.

12. Der medizinische Draht nach einem der Ansprüche 8 bis 11, **dadurch gekennzeichnet, dass** der Windungsabstand des Spulendrahtes des vorderen Endstücks (2b) in einem Abschnitt, an welches das proximale Ende (2h) des thermisch lösbaren Verbindungselements (2c) gebunden und fixiert ist, über 110% ist, wenn die benachbarten Spulendrähte, die miteinander in direktem Kontakt stehen, als 100% festgelegt sind.

## Revendications

1. Fil médical comprenant un élément d'extrémité avant (2b) étant formé d'un fil électroconducteur en forme de bobine avec une extrémité proximale (2h) et une extrémité distale, un fil de poussée électroconducteur (2a) relié à l'extrémité proximale (2h) de l'élément d'extrémité avant (2b) et un élément d'insertion (2e) connecté par un élément de connexion thermosoluble (2c) à l'extrémité distale de l'élément d'extrémité avant (2b), ledit fil médical séparant ledit élément d'insertion par le chauffage dudit élément de connexion par un courant électrique, une partie de la section du fil de bobine dudit élément d'extrémité avant étant prévue de ne pas être en contact rapproché avec une section de fil de bobine voisine vue en coupe, le fil de bobine dudit élément d'extrémité avant ayant au moins plus que deux pas différents et ledit fil médical étant prévu avec un fil de sécurité (2d) disposé à l'intérieur de l'enroulement de la bobine dudit élément d'extrémité avant et s'étendant entre l'extrémité proximale (2h) à l'extrémité distale et reliant les deux extrémités **caractérisé en ce que** l'extrémité distale du fil de sécurité est reliée à une circonférence intérieure du fil de bobine constituant l'élément d'extrémité avant.

2. Fil médical selon la revendication 1, dans lequel une extrémité du fil de sécurité est une extrémité distale du fil de sécurité.

3. Fil médical selon l'une des revendications 1 ou 2, **caractérisé en ce qu'**une extrémité proximal du fil de sécurité est liée aux deux ou à un de l'extrémité proximale (2h) dudit élément d'extrémité avant et du fil de poussée électroconducteur (2a).

4. Fil médical selon l'une des revendications 1 à 3, **caractérisé en ce que** la connexion de l'extrémité distale du fil de sécurité à la circonférence intérieure (2g) du fil de bobine constituant l'élément d'extrémité avant (2b) est du soudage, du brasage ou du collage.

5. Fil médical selon l'une des revendications 1 à 4, **caractérisé en ce que** ledit fil de sécurité est un fil rond ou un fil carré.

6. Fil médical selon l'une des revendications 1 à 5, **caractérisé en ce que** ledit élément d'extrémité avant et le fil de sécurité (2d) incluent un métal biocompatible.

7. Fil médical selon l'une des revendications 1 à 6, **caractérisé en ce que** ledit élément d'extrémité avant est ledit fil de sécurité incluent au moins un type de métal sélectionné parmi le groupe consistant en de la platine, du palladium, d'or, du tungstène, du tantale, de l'iridium, des alliages de l'acier inoxydable, des alliages hyperélastiques et des alliages de ces métaux.

8. Fil médical selon l'une des revendications 1 à 7, **caractérisé en ce que** ledit élément d'extrémité avant inclut une unité d'électrode (2j) pour la fusion à chaud dudit élément de connexion thermosoluble par un courant électrique et une portion à qui l'extrémité proximale (2h) dudit élément de connexion thermosolbule (2c) est liée et fixée.

9. Fil médical selon la revendication 8, **caractérisé par** les fils de bobine voisins réalisant l'unité d'électrode (2j) de l'élément d'extrémité avant (2b) qui sont réciproquement en contact rapproché.

10. Fil médical selon la revendication 9, **caractérisé par** ledit élément de connexion thermosotuble en contact rapproché avec la circonférence intérieure (2g) du fil de bobine réalisant l'unité d'électrode (2j) de l'élément d'extrémité avant (2b).

11. Fil médical selon l'une des revendications 1 à 10, **caractérisé en ce qu'**un pas du file de bobine d'une deuxième section où l'extrémité distale du fil de sécurité est connectée à la circonférence intérieure (2g) du fil de bobine réalisant l'élément d'extrémité avant (2b) est réglé plus grand que le pas du fil de bobine d'une troisième section.

12. Fil médical selon l'une des revendications 8 à 11, **caractérise en ce que** le pas du fil de bobine de l'élément d'extrémité avant (2b) dans une portion à qui l'extrémité proximale (2h) dudit élément de connexion thermosoluble (2c) est liée et fixée est plus que 110 % si des fils de bobine voisins en contact rapproché réciproque sont mis en 100 %.
